# FASCICULE DE BREVET EUROPEEN

(11) **EP 1 251 132 B1**
(45) Date de publication et mention de la délivrance du brevet: **16.06.2004**
(21) Numéro de dépôt: 02290946.9
(22) Date de dépôt: 16.04.2002
(51) Int. Cl.: C07D 513/04

(54) **Procédé de synthèse de la (3aS)-5,5-dioxo-2,3,3a,4-tétrahydro-1H-pyrrolo (2,1-c)(1,2,4)benzothiadiazine**
Verfahren zur Herstellung von (3aS)-5,5-Dioxo-2,3,3a,4-tetrahydro-1H-pyrrolo[2,1-c][1,2,4]benzothiadiazin
Synthesis of (3aS)-5,5-dioxo-2,3,3a,4-tetrahydro-1H-pyrrolo[2,1-c][1,2,4]benzothiadiazine

(30) Priorité: 18.04.2001 FR 0105226
(43) Date de publication de la demande: 23.10.2002
(73) Titulaire: Les Laboratoires Servier, 92200 Neuilly-sur-Seine (FR)
(72) Inventeur: Foucher, Elsa, 76110 Sausseuzemare-en-Caux (FR); Thominot, Gilles, 76640 Normanville (FR); Lecouve, Jean-Pierre, 76600 Le Havre (FR); Ramsden, James Andrew, Cambridge CB4 2QP (GB); Cobley, Christopher James, Histon, Cambridge 9JU (GB)

(56) Documents cités:
- EP-A- 0 692 484
- DESOS, P. ET AL.: "Enantioselective synthesis of a pyrrolo-benzothiadiazine derivative" BIOORGANIC & MEDICINAL CHEMISTRY LETTERS, vol. 6, no. 24, 1996, pages 3003-3008, XP001041810 OXFORD, GB ISSN: 0960-894X

## Description

La présente invention concerne un nouveau procédé de synthèse industrielle de la (3aS)-5,5-dioxo-2,3,3a,4-tétrahydro-1*H*-pyrrolo[2,1-c][1,2,4]benzothiadiazine de formule (I) : 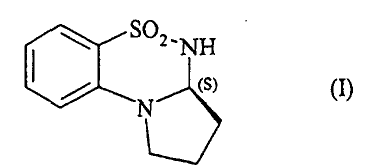 et de ses sels d'addition à un acide pharmaceutiquement acceptable.

Le composé de formule (I), ainsi que ses sels, possèdent une puissante activité facilitatrice de l'activation induite par l'acide glutamique au niveau des récepteurs AMPA, ce qui les rend utiles dans le traitement et la prévention des pathologies liées au dysfonctionnement de la neurotransmission glutamatergique telles que les désordres mnémocognitifs associés à l'âge et aux syndromes anxieux ou dépressifs, les déficits mnésiques des maladies neurodégénératives progressives, ainsi que les séquelles des maladies neurodégénératives aiguës.

Le composé de formule (I), son utilisation en thérapeutique ainsi qu'une méthode de préparation ont été décrits dans le brevet EP 0 692 484.

Compte-tenu de l'intérêt pharmaceutique de ce composé, et du fait que seul l'isomère (S) possède une activité facilitatrice sur le courant AMPA, il était primordial de pouvoir le préparer avec un procédé de synthèse performant, permettant l'obtention sélective de l'isomère (S) avec un bon rendement et une excellente pureté, et facilement transposable à l'échelle industrielle.

Deux méthodes de préparation du composé de formule (I) sont déjà connues. Toutefois, ces procédés ne sont pas utilisables à l'échelle industrielle :
- Le brevet EP 0 692 484 décrit l'accès au composé de formule (I) par réduction non énantiosélective de la 5,5-dioxo-2,3-dihydro-1*H*-pyrrolo[2,1-c][1,2,4]benzothiadiazine au moyen de borohydrure de sodium, suivie de la séparation du mélange racémique obtenu par chromatographie HPLC préparative sur phase chirale.
   Or, ce mode de séparation n'est pas envisageable à l'échelle industrielle, en raison de sa très faible productivité.
- La publication Bioorg. Med. Chem. Lett. 1996, 6, 3003 décrit l'accès au composé de formule (I) par réduction de la 5,5-dioxo-2,3-dihydro-1*H*-pyrrolo[2,1-c][1,2,4]benzothiadiazine au moyen d'un complexe chiral d'hydrure de lithium aluminium.
   Toutefois, la faible énantiosélectivité de la réduction rend nécessaire un enrichissement fastidieux pour obtenir le composé de formule (I) sous sa forme optiquement pure.

La demanderesse a présentement mis au point un procédé de synthèse industrielle du composé de formule (I) par hydrogénation catalytique énantiosélective de la 5,5-dioxo-2,3-dihydro-1*H*-pyrrolo[2,1-c][1,2,4]benzothiadiazine, permettant d'obtenir directement l'isomère (S) avec un rendement et une pureté chimique et énantiomérique excellents.

Plus spécifiquement, la présente invention concerne un procédé de synthèse industrielle du composé de formule (I) caractérisé en ce que l'on hydrogène la 5,5-dioxo-2,3-dihydro-1*H*-pyrrolo[2,1-c][1,2,4]benzothiadiazine de formule (II) : 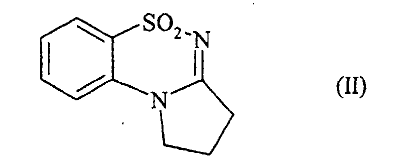 en présence du catalyseur (R) BINAP RuCl₂ (R,R) DPEN de formule (III) : 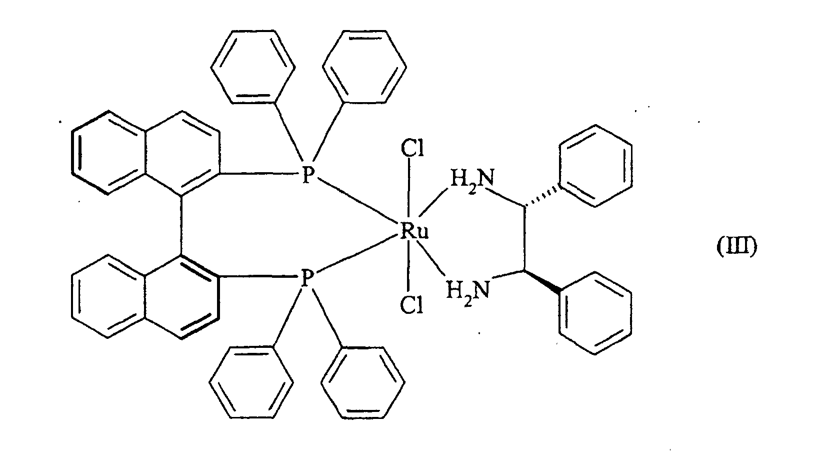 en quantité comprise entre 0,4 et 2 mmoles par mole de composé de formule (II).
- dans un mélange de toluène et d'isopropanol dans lequel la proportion de toluène est comprise entre 10 et 90 % en volume, préférentiellement entre 70 et 80 % en volume,
- sous une pression d'hydrogène comprise entre 4 et 25 bars, préférentiellement entre 10 et 15 bars,
- à une température comprise entre 40 et 90°C, préférentiellement entre 65 et 75°C,
- et en présence d'une base telle que, par exemple, le tert-butanolate de potassium ou de sodium en solution dans un solvant alcoolique tel que, par exemple, le tert-butanol ou l'isopropanol,
   en quantité comprise entre 0,8 et 1,5 mole par mole de composé de formule (II), préférentiellement entre 1 et 1,2 mole par mole de composé de formule (II),
   pour conduire directement, après isolement puis recristallisation, au composé de formule (I) avec un excès énantiomérique supérieur à 80 %.

L'exemple ci-dessous illustre l'invention, mais ne la limite en aucune façon.

La pureté chimique du composé de formule (I) a été déterminée par chromatographie HPLC sur colonne HYPERSIL BDS C18, en utilisant comme éluant un mélange eau/acétonitrile 25/75.
(Détecteur : 210 nm ; Four : 30°C ; Débit 1 ml/mn)

La pureté énantiomérique du composé de formule (I) a été déterminée par chromatographie HPLC sur colonne CHIRALPACK AS (Daicel), en utilisant comme éluant un mélange éthanol/heptane 70/30.
(Détecteur : 212 nm ; Four : 25°C ; Débit 1 ml/mn)

### Abréviations :

*BINAP : 2,2'-(bis(diphénylphosphino))-1,1'-binaphtyle*
*DPEN : diphényléthylènediamine*

### EXEMPLE : (3aS)-5,5-Dioxo-2,3,3a,4-tétrahydro-1H-pyrrolo[2,1-c][1,2,4] benzothiadiazine

La réaction est effectuée dans un autoclave.
A 40 g de 5,5-dioxo-2,3-dihydro-1*H*-pyrrolo[2,1-c][1,2,4]benzothiadiazine en solution dans 450 ml de toluène préalablement dégazé à l'azote sont ajoutés 90,5 mg de catalyseur (R)-BINAP RuCl₂ (R,R)-DPEN de formule (III), puis une solution, préalablement chauffée à 50°C, de tert-butanolate de potassium (20,2 g) dans l'isopropanol (150 ml).
Après une purge à l'azote, le milieu est chauffé à 70 °C sous agitation, puis une pression d'hydrogène de 15 bars est appliquée pendant 20 heures, toujours sous agitation.
Après décompression et purge à l'azote, le milieu réactionnel est mis à sec, puis le résidu obtenu est recristallisé dans l'acétone.
Le composé de formule (I) est ainsi obtenu directement avec un rendement quantitatif, une pureté chimique supérieure à 90 % et un excès énantiomérique de 83 %.

## Revendications

1. Procédé de synthèse industrielle de la (3aS)-5,5-dioxo-2,3,3a,4-tétrahydro-1*H*-pyrrolo[2,1-c][1,2,4]benzothiadiazine de formule (I) : 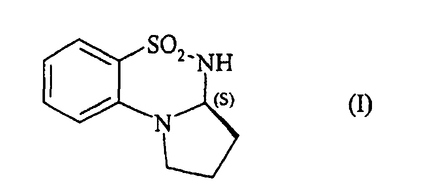 **caractérisé en ce que** l'on hydrogène la 5,5-dioxo-2,3-dihydro-1*H*-pyrrolo[2,1-c][1,2,4]benzothiadiazine de formule (II) : 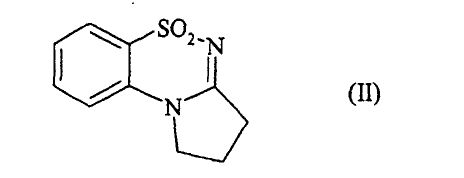 en présence du catalyseur (R) BINAP RuCl₂ (R,R) DPEN de formule (III) : 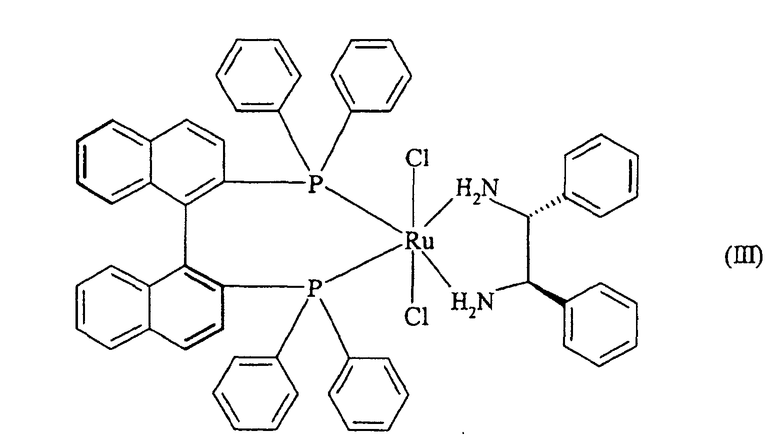 en quantité comprise entre 0,4 et 2 mmoles par mole de composé de formule (II).
- dans un mélange de toluène et d'isopropanol dans lequel la proportion de toluène est comprise entre 10 et 90 % en volume,
- sous une pression d'hydrogène comprise entre 4 et 25 bars,
- à une température comprise entre 40 et 90°C,
- et en présence d'une base telle que, par exemple, le tert-butanolate de potassium ou de sodium en solution dans un solvant alcoolique tel que, par exemple, le tert-butanol ou l'isopropanol,
en quantité comprise entre 0,8 et 1,5 mole par mole de composé de formule (II),
pour conduire directement, après isolement puis recristallisation, au composé de formule (I) avec un excès énantiomérique supérieur à 80 %.

2. Procédé selon la revendication 1 **caractérisé en ce que** la proportion de toluène dans le mélange toluène/isopropanol est comprise entre 70 et 80 % en volume.

3. Procédé selon l'une quelconque des revendications 1 ou 2 **caractérisé en ce que** la pression d'hydrogène est comprise entre 10 et 15 bars.

4. Procédé selon l'une quelconque des revendications 1 à 3 **caractérisé en ce que** la température d'hydrogénation est comprise entre 65 et 75°C.

5. Procédé selon l'une quelconque des revendications 1 à 4 **caractérisé en ce que** la quantité de base utilisée est comprise entre 1 et 1,2 mole par mole de composé de formule (II).

## Patentansprüche

1. Verfahren zur technischen Herstellung von (3aS)-5,5-Dioxo-2,3,3a,4-tetrahydro-1*H*-pyrrolo[2,1-c][1,2,4]benzothiadiazin der Formel (I): 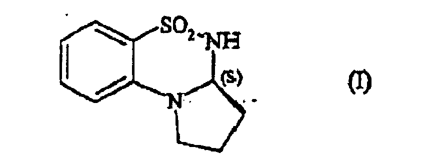 **dadurch gekennzeichnet, daß** man 5,5-Dioxo-2,3-dihydro-1*H*-pyrrolo[2,1-c][1,2,4]-benzothiadiazin der Formel (II): 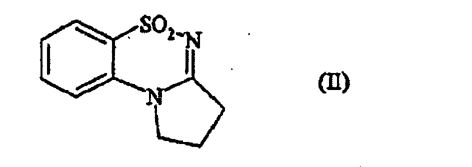 in Gegenwart eines (R) BINAP RuCl₂ (R,R) DPEN-Katalysators der Formel (III): 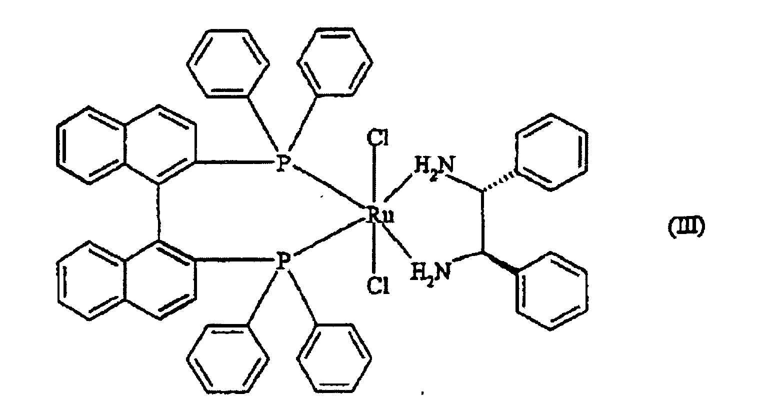 in einer Menge zwischen 0,4 und 2 mMol pro Mol der Verbindung der Formel (II)
- in Gegenwart einer Mischung aus Toluol und Isopropanol, in der der Toluol-Anteil zwischen 10 und 90 Volumen-% liegt,
- unter einem Wasserstoffdruck zwischen 4 und 25 bar,
- bei einer Temperatur zwischen 40 und 90°C,
- und in Gegenwart einer Base, wie beispielsweise Kalium-tert.-butanolat oder Natrium-tert.-butanolat, in Lösung in einem alkoholischen Lösungsmittel, wie beispielsweise tert.-Butanol oder Isopropanol,
in einer Menge zwischen 0,8 und 1,5 Mol pro Mol der Verbindung der Formel (II), hydriert,
so daß man direkt, nach der Isolierung und der Umkristallisation, die Verbindung der Formel (I) mit einem Enantiomeren-Überschuß von mehr als 80 % erhält.

2. Verfahren nach Anspruch 1, **dadurch gekennzeichnet, daß** der Toluol-Anteil in der Toluol/Isopropanol-Mischung zwischen 70 und 80 Volumen-% liegt.

3. Verfahren nach einem der Ansprüche 1 oder 2, **dadurch gekennzeichnet, daß** der Wasserstoffdruck zwischen 10 und 15 bar liegt.

4. Verfahren nach einem der Ansprüche 1 bis 3, **dadurch gekennzeichnet, daß** die Temperatur der Hydrierung zwischen 65 und 75°C liegt.

5. Verfahren nach einem der Ansprüche 1 bis 4, **dadurch gekennzeichnet, daß** die verwendete Menge der Base zwischen 1 und 1,2 Mol pro Mol der Verbindung der Formel (II) liegt.

## Claims

1. Process for the industrial synthesis of (3aS)-5,5-dioxo-2,3,3a,4-tetrahydro-1*H*-pyrrolo-[2,1-c][1,2,4]benzothiadiazine of formula (I) : 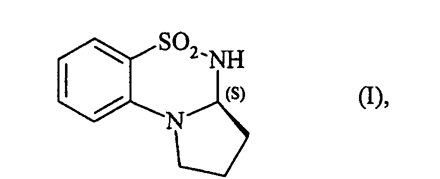 **characterised in that** 5,5-dioxo-2,3-dihydro-1*H*-pyrrolo[2,1-c][1,2,4]benzothiadiazine of formula (II) : 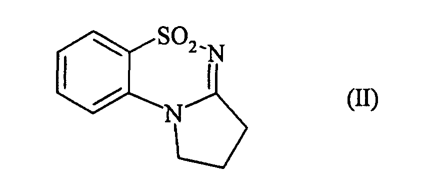 is hydrogenated in the presence of the catalyst (R)-BINAP RuCl₂ (R,R)-DPEN of formula (III) : 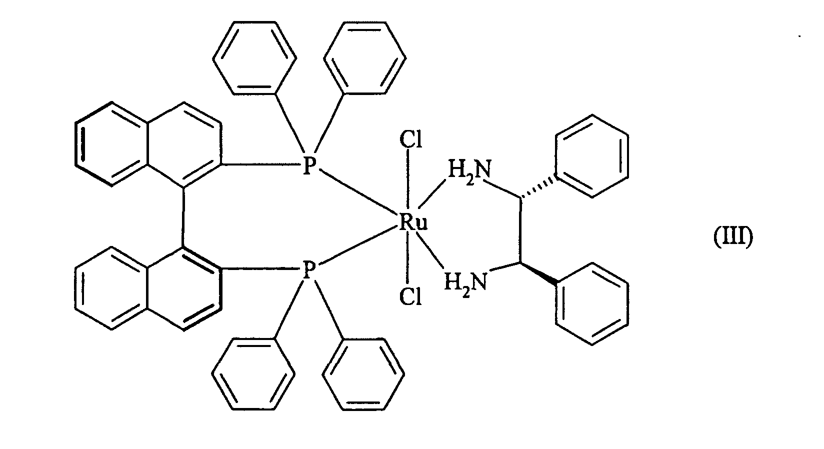 in an amount of from 0.4 to 2 mmol per mol of compound of formula (II),
- in a mixture of toluene and isopropanol wherein the proportion of toluene is from 10 to 90 % by volume,
- under hydrogen pressure of from 4 to 25 bar,
- at a temperature of from 40 to 90°C,
- and in the presence of a base such as, for example, potassium or sodium tert-butanolate dissolved in an alcoholic solvent such as, for example, tert-butanol or isopropanol,
in an amount of from 0.8 to 1.5 mol per mol of compound of formula (II),
to yield directly, after isolation and then recrystallisation, the compound of formula (I) having an enantiomeric excess of more than 80 %.

2. Process according to claim 1, **characterised in that** the proportion of toluene in the toluene/isopropanol mixture is from 70 to 80 % by volume.

3. Process according to either claim 1 or claim 2, **characterised in that** the hydrogen pressure is from 10 to 15 bar.

4. Process according to any one of claims 1 to 3, **characterised in that** the hydrogenation temperature is from 65 to 75°C.

5. Process according to any one of claims 1 to 4, **characterised in that** the amount of base used is from 1 to 1.2 mol per mol of compound of formula (II).
